# EUROPÄISCHE PATENTSCHRIFT

(11) **EP 4 251 685 B1**
(45) Veröffentlichungstag und Bekanntmachung des Hinweises auf die Patenterteilung: **29.01.2025**
(21) Anmeldenummer: 21824506.6
(22) Anmeldetag: 29.11.2021
(51) Int. Cl.: C08J 11/14, C08J 11/28, C07C 209/62

(54) **VERFAHREN ZUM ABBAU VON POLYURETHAN**
PROCESS FOR THE DECOMPOSITION OF POLYURETHANE
PROCÉDÉ DE DÉCOMPOSITION DE POLYURÉTHANE

(30) Priorität: 27.11.2020 DE 102020131581
(43) Veröffentlichungstag der Anmeldung: 04.10.2023
(73) Patentinhaber: NEVEON GERMANY GMBH, 65203 Wiesbaden (DE)
(72) Erfinder: BETTINGER, Herbert, 66606 St. Wendel (DE)
(74) Vertreter: Rösler Rasch van der Heide & Partner
(86) Internationale Anmeldenummer: PCT/EP2021/083434
(87) Internationale Veröffentlichungsnummer: WO 2022/112581

(56) Entgegenhaltungen:
- WO-A1-2022/093523
- US-A- 4 196 148
- US-A- 5 208 379
- MOTOKUCHO SUGURU ET AL: "Hydrolysis of aromatic polyurethane in water under high pressure of CO 2", JOURNAL OF POLYMER SCIENCE PART A: POLYMER CHEMISTRY, vol. 55, no. 12, 15 June 2017 (2017-06-15), US, pages 2004 - 2010, XP055893370, ISSN: 0887-624X, Retrieved from the Internet <URL:https://api.wiley.com/onlinelibrary/tdm/v1/articles/10.1002%2Fpola.28576> DOI: 10.1002/pola.28576
- MIR MOHAMMAD ALAVI NIKJE ET AL: "Polyurethane Waste Reduction and Recycling: From Bench to Pilot Scales", DESIGNED MONOMERS AND POLYMERS, vol. 14, no. 5, 1 January 2011 (2011-01-01), pages 395 - 421, XP055520968, DOI: 10.1163/138577211X587618

## Beschreibung

Die Erfindung betrifft ein Verfahren zum Abbau von Polyurethan sowie ein über dieses Verfahren erhältliches Verfahrensmedium.

Polyurethane finden aufgrund ihrer vielen einstellbaren Eigenschaften weite Verwendung für Produkte, die in der Industrie oder in Haushalten benutzt werden. Beispiele für derartige Produkte sind Schaumstoffe, Lacke, Klebstoffe, Vergussmassen, Schläuche, Dichtungen, Bodenbeläge, Matratzen, Autoteile, Teile von Sportgeräten, Teile von Schuhen, und dergleichen.

Dementsprechend fällt ein hoher Anteil an Polyurethan-Abfällen an, wenn die entsprechenden Produkte beschädigt werden oder das Ende ihrer Lebensdauer erreicht haben.

In der Vergangenheit wurden daher mehrere Lösungen vorgeschlagen, um Polyurethan-Abfälle aufzuarbeiten. Mehrere Patentschriften betreffen Verfahren, in denen Polyurethan-Abfälle in aliphatischen Diolen gelöst werden, beispielsweise die US-Patente 4,044,04, 3,632,530 und 4,162,995 sowie die deutsche Offenlegungsschrift 2304444. Das US-Patent 4,316,992 schlägt ein Lösen in hochsiedendem gesättigten Alkohol vor, und das US-Patent 4,039,568 ein Lösen in Gegenwart verschiedener Alkoholate. Nachteilig bei all diesen Verfahren ist der Umstand, dass dafür Kohlenwasserstoffe in Form von Diolen benötigt werden. Motokucho et al. Hydrolysis of aromatic polyurethane in water under high pressure of CO2, Journal of Polymer Science Part A: Polymer Chemistry, 2017, Bd. 55, Nr. 12, Seiten 2004-2010, offenbart ein Verfahren zum Abbau von Polyurethan mittels Hydrolyse unter Überdruck und Anwesenheit von Kohlenstoffdioxid.

Aufgabe der Erfindung besteht darin, alternative Lösungen bereitzustellen, bei denen vorzugsweise die Umwelt weniger belastende Substanzen zum Einsatz kommen.

Die Erfindung ergibt sich aus den Merkmalen der unabhängigen Ansprüche. Vorteilhafte Weiterbildungen und Ausgestaltungen sind Gegenstände der abhängigen Ansprüche.

Die Aufgabe ist in einem ersten Aspekt gelöst durch ein Verfahren zum Abbau von Polyurethan, wobei man ein Polyurethan enthaltendes Material in Gegenwart einer 1 bis 45 Massenprozent Harnstoff enthaltenden wässrigen Lösung bei Überdruck auf eine Temperatur von 190 °C bis 250 °C erhitzt. Zu Beginn des Verfahrens liegt Polyurethan enthaltendes Material in oder zusammen mit der wässrigen, Harnstoff enthaltenden Lösung vor. Nach Ablauf der gewählten Behandlungsdauer ist der ursprünglich in fester Form vorliegende Polyurethan-Anteil des Polyurethan enthaltenden Materials teilweise oder vollständig in Substanzen übergeführt, die sich in einem flüssigen Verfahrensmedium befinden, welches zurückzuführen ist auf die zu Beginn des Verfahrens vorliegende Harnstoff enthaltende wässrige Lösung. Das Verfahrensmedium enthält gegebenenfalls noch Feststoffe, beispielsweise Feststoffe, die im festen Polyurethan eingebettet oder daran angelagert waren, oder von Polyurethan abweichende Kunststoffe. Das flüssige Verfahrensmedium enthält Abbauprodukte und/oder weitere Folgeprodukte, die auf das ursprünglich vorliegende Polyurethan zurückzuführen sind. Durch die Überführung in das flüssige Verfahrensmedium ergibt sich vorteilhaft die Möglichkeit, ursprünglich festes Polyurethan in eine leichter handhabbare Form zu überführen, und gegebenenfalls aus der erhaltenen flüssigen Verfahrensmedium Wertstoffe zu isolieren oder diese in weiteren Reaktionen nutzbringend umzusetzen.

Unter Überdruck wird jeder Druck verstanden, der höher ist als der atmosphärische Umgebungsdruck. Gemäß einer Ausführungsform erfolgt das Erhitzen bei einem Druck von 1,05 bar bis 100 bar. Beispiele für Druckbereiche sind 10 bar bis 45 bar, beispielsweise 12 bar bis 40 bar, oder 25 bar bis 35 bar. Mit dem Ziel einer einfachen Prozessführung ist der Überdruck ein sich bei dem Erhitzen einstellender Gleichgewichtsdruck, beispielsweise der bei einem Erhitzen in einem Druckgefäß sich einstellende Gleichgewichtsdruck.

Vorzugsweise wird das Verfahren innerhalb eines Zeitraums von 20 Minuten bis 240 Minuten durchgeführt, insbesondere 45 Minuten bis 240 Minuten, 90 Minuten bis 240 Minuten, beispielsweise 120 Minuten bis zu 240 Minuten. Ein Beispiel für einen bevorzugten Zeitraum ist der Zeitraum von 30 Minuten bis 180 Minuten, beispielsweise der Zeitraum zwischen 40 Minuten und 180 Minuten (40 Minuten bis 180 Minuten). Die Reaktionstemperatur kann dabei konstant bei einem Wert innerhalb des angegebenen Temperaturbereichs bleiben, oder verschiedene Werte innerhalb des angegebenen Temperaturbereichs annehmen, beispielsweise, um durch Temperaturregelung einen vorgegebenen Zielwert zu erreichen. Der Zeitraum ist vorzugsweise ein einziger zusammenhängender Zeitraum, kann sich jedoch auch kumulativ aus voneinander getrennten Zeiträumen, in denen die vorgesehenen Temperaturbedingungen eingehalten werden, zusammensetzen. Im Rahmen von aufwendigen Versuchen wurde festgestellt, dass bei einer Temperatur von 245 °C und einer Zeitdauer von 240 Minuten erste Anzeichen für die Bildung von Feststoffen auftraten. Unter Berücksichtigung einer vorgegebenen Dauer der Temperaturbehandlung von 240 min wird als praktikable Obergrenze für die Temperatur daher eine Temperatur von 250°C angesehen.

Mit dem Verfahren wird die Möglichkeit eröffnet, einen Abbau von Polyurethan unter Verwendung von Harnstoff zu erreichen, also einer Substanz, die in der Umwelt natürlich vorkommt und dementsprechend auch natürlich abbaubar ist.

Bei dem Polyurethan enthaltenden Material kann es sich um Material handeln, das zusätzlich zum Polyurethan und abgegrenzt davon eine Komponente aus einem Nicht-Polyurethan enthält, beispielsweise Polyurethan in Kombination mit einer Komponente aus einem weiteren Kunststoff, wie etwa Partikel aus Styrol-Acryl-Nitril, einer Komponente aus einem Metall, einer Komponente aus einem Glas, oder einer Komponente aus einem anorganischen Material, beispielsweise Kalziumcarbonat. Nicht-beschränkte Beispiele hierfür sind Polyurethan-Gegenstände, die Befestigungselemente aus Metall enthalten, oder Verbundwerkstoffe aus Polyurethan und einem weiteren Kunststoff, beispielsweise Teile von Kühlschränken, die Polyurethan als wärmedämmende Schicht enthalten und andere Kunststoffe als Abdeckschicht. Polyurethan enthaltendes Material kann jedoch auch aufgefasst werden als aus Polyurethan bestehendes Material. Das Polyurethan selbst kann reines Polyurethan sein, oder ein Polyurethan, in dem weitere Substanzen enthalten sind, beispielsweise Weichmacher, mikrobizide Substanzen, Antioxidantien, Stabilisatoren, beispielsweise gegenüber UV-Licht, Flammschutzmittel, Farbstoffe oder Reste von Polymerisationsinitiatoren. Unter dem Begriff "Polyurethan enthaltendes Material" kann auch eine Mischung verschiedener Polyurethan enthaltender Materialien umfasst sein, beispielsweise Materialien, die jeweils unterschiedliche Arten von Polyurethan enthalten.

Der Abbau des Polyurethans kann teilweise erfolgen, kann jedoch je nach gewählten Verfahrensparametern über Zwischenstufen, bei denen das Polyurethan in zerkleinerter Form vorliegt, bis zum vollständigen Abbau des in dem Material enthaltenen Polyurethans führen, so dass das Polyurethan nicht mehr als Feststoff vorliegt.

Unter Polyurethan wird wie fachüblich ein Polymer verstanden, das als charakteristische Gruppe die Urethangruppe gemäß der nachstehenden Formel (I) aufweist:

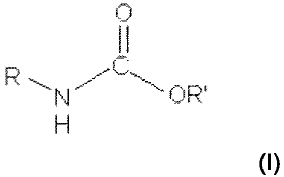

Polyurethane sind im Allgemeinen erhältlich durch die Polyaddition von zwei- oder höherwertigen Alkoholen der Formel (II)

HO-R'-OH (II),

wobei R' für einen niedermolekularen oder selbst bereits polymeren aliphatischen oder aromatischen Rest steht, der gegebenenfalls wenigstens eine weitere Hydroxylgruppe umfasst,
mit Diisocyanaten der allgemeinen Formel (III)

   O=C=N-R-N=C=O (III),
wobei R die gleiche Bedeutung hat wie R'.

Die Chemie und die technische Herstellung und Verarbeitung von Polyurethanen ist dem Fachmann allgemein bekannt und beispielsweise beschrieben in Ullmann's Encyclopedia of Industrial Chemistry, 6th Completely Revised Edition, Wiley-VHC Verlag GmbH & Co. KGaA, Weinheim, Deutschland, 2003, Band 28, Seite 667-722. Der größte Teil der Polyurethan-Schaumstoffe (nachfolgend auch als PUR-Schaumstoffe bezeichnet) wird auf der Basis aromatischer Isocyanate hergestellt. Die wichtigsten Vertreter dieser Gruppe sind Gemische aus den Isomeren 2,4-Toluoldiisocyanat und 2,6-Toluoldiisocyanat (TDI), sowie Gemische aus Isomeren von Diphenylmethandiisocyanat (MDI), wofür als nicht-beschränkende Beispiele Diphenylmethan-2,2'-diisocyanat (2,2'-MDI), Diphenylmethan-2,4'-diisocyanat (2,4'-MDI) und Diphenylmethan-4,4'-diisocyanat (4,4'-MDI) genannt werden können, und präpolymerisiertem MDI. TDI 80, ist das bedeutendste Diisocyanat bei der Weichschaumstoffproduktion. TDI 65 kommt bei vielen Weichschaumstoffen, insbesondere auch bei Ester-Schaumstoffen zum Einsatz. In beiden Bezeichnungen steht die Zahl 80 beziehungsweise 65 für den massenprozentualen Anteil des hochreaktiven Isomers 2,4-Toluoldiisocyanat, die Differenz zu 100 Massenprozent wird vom Isomer 2,6-Toluoldiisocyanat bereitgestellt. Als Polyolkomponenten kommen vorzugsweise Polyether, Polyester oder Diamine zum Einsatz. Die Eigenschaften lassen sich zudem auch noch durch Zugabe von Stabilisatoren wie Silicon-Polyether-Copolymeren, Epoxiden, Benzophenon und weiteren Substanzen verändern. Auch Additive können die Eigenschaften des Schaumes variieren. So wird beispielsweise Phosphorsäureester als Flammschutzmittel verwendet. Auch mechanische Verstärkungsmittel wie beispielsweise Karbonfasern können in die Schaumstoffe eingebaut werden, und stellen Beispiele für Nicht-Polyurethane dar, die als weitere Komponenten in dem Polyurethan enthaltenden Material enthalten sein können. Ebenso können Füllstoffe, wie zum Beispiel Calciumcarbonat verwendet werden.

Bei dem Polyurethan kann es sich um geschäumtes Polyurethan oder porenfreies Polyurethan handeln, wobei nicht-beschränkende Beispiele für porenfreies Polyurethan Schläuche aus Polyurethan oder Dichtungsmassen aus Polyurethan sind.

Gemäß einer besonderen Ausgestaltung des Verfahrens umfasst das Polyurethan (PUR), Polyurethan-Weichschaum (PUR-Weichschaum), Polyurethan-Hartschaum (PUR-Hartschaum), Schredderleichtfraktion oder Mischung zweier oder mehrerer Vertreter davon, oder besteht insbesondere daraus. Der Begriff der Schredderleichtfraktion ist dem Fachmann der Abfallwirtschaft bekannt und beschreibt im Allgemeinen eine heterogene Mischung, die aus verschiedenen Kunststoffen, organischen und anorganischen Materialien bestehen kann, wobei die konkrete Zusammensetzung von der Art des geschredderten Abfalls abhängt. Ein Beispiel für eine Schredderleichtfraktion fällt beim Recycling von Kühlschränken an, bei denen die Ausschäumung von Hohlräumen aus PUR-Hartschaumstoff, gegebenenfalls unter Beimischung anderer Kunststoffe besteht, etwa Kunstoffen, die auf Durchführungen von Kabeln zurückzuführen sind. Schredderleichtfraktion aus dem Kühlschrankrecycling kann weiterhin mineralische Anteile enthalten, etwa aufgrund von im Polyurethan enthaltenen Füllstoffen.

Nicht-beschränkende Beispiele für Quellen für Polyurethan enthaltendes Material sind Produktionsabfälle der Matratzenindustrie, der Automobilindustrie, der Bauindustrie, der Möbelindustrie, der Schuhindustrie, der Elektroindustrie und der Sport/Freizeitindustrie, beziehungsweise defekte oder nicht mehr benutzte Produkte dieser Industrien, wie etwa Matratzen; Karosserie- oder andere Fahrzeugteile wie etwa Stoßfänger, Armaturentafeln, Kopfstützen, Armlehnen oder Teppiche; Wandpaneele oder Rohrdämmungen; Möbel oder Möbelteile; Schuhe oder Schuhteile, wie etwa Sohlen oder Schuhkappen; Kabelummantelungen, Stecker, Steckerleisten oder Teile davon; oder Sportgeräte wie Snowboards oder Laufrollen von Rollschuhen.

Im Rahmen des erfindungsgemäßen Verfahrens können als Polyurethan enthaltendes Material insbesondere Polyurethan-Schaumstoffe (nachfolgend auch als PUR-Schaumstoffe bezeichnet) verwendet werden, die ausgewählt sind unter folgenden Zusammensetzungen: PUR-Weichschaumstoffen basierend auf nichtreaktiven Polyetherpolyolen (sogenannten Standardpolyetherpolyolen) mit Molekulargewichten um 3000 g/Mol, gefüllt (mit SAN-Copolymeren, d.h. Styrol-Acrylnitril-Copolymeren) oder ungefüllt; PUR-Weichschaumstoffen basierend auf reaktiven High Resilience Polyetherpolyolen mit Molekulargewichten von mehr als 3000 g/Mol, gefüllt (mit SAN-Copolymeren oder PHD, d.h. Polyharnstoff-Dispersion) oder ungefüllt; PUR-Weichschaumstoffen basierend auf reaktiven 6-funktionellen Polyetherpolyolen; PUR-Weichschaumstoffen basierend auf Hypersoft Polyetherpolyolen; PUR-Weichschaumstoffen basierend auf Polyetherpolyolen und Mischungen daraus, die zu viskoelastischen Schaumstoffen führen und als Komponente auch PEG (Polyethylenglycol) enthalten können; PUR-Weichschaumstoffen basierend auf Polyesterpolyolen, gefüllt oder ungefüllt; PUR-Weichschaumstoffen basierend auf vorgenannten Polyolmischungen in Verbindung mit TDI oder MDI; und PUR-Hart- oder Halbhartschaumstoffen basierend auf vorgenannten Polyolmischungen in Verbindung mit TDI oder MDI, sowie Integral-Hartschaumstoffen bestehend aus vorgenannten Komponenten.

Gemäß besonderen Weiterbildungen umfassen die PUR-Schaumstoffe einen oder mehrere Vertreter aus der Produktfamilie der Weichschaumstoffe und/oder einen oder mehrere Vertreter aus der Produktfamilie der Hartschaumstoffe.

Weichschaumstoffe umfassen Kunststoffe wie Standardpolyether-Schaumstoffe, High-Resilience Polyether- Schaumstoffe, Combustion modified-Polyether-Schaumstoffe (CME), Combustion modified high resilience-Polyether-Schaumstoffe (CMHR), Viskoelastische Polyether-Schaumstoffe und Polyester-Schaumstoffe. Beispielhafte Zusammensetzungen sind dem Fachmann bekannt und werden zum Beispiel beschrieben in den Druckschriften DE 3630225 C2, US 3,905,924 und DE 10 2007 051 089 A1.

Vorzugsweise umfassen diese Weichschaumstoffe folgende Bestandteile oder bestehen aus Kombinationen zweier oder mehrerer Vertreter daraus:
Isocyanate:
   2,4- und/oder 2,6-Toluylendiisocyanat (TDI) sowie beliebige Gemische dieser Isomere;
   4,4'- und/oder 2,2'- Diphenylmethandiisocyanate (MDI) sowie beliebige Gemische dieser Isomere;
   Polymer MDI ("Roh"-MDI) sowie mit mehrwertigen Polyolen (vorzugsweise zwei- und/oder dreiwertigen Polyethern) präpolymerisiertes MDI; und/oder
   beliebige Gemische von TDI und MDI aus vorgenannten Isomeren und Ausbildungen.
Polyole:
   Polyether-Polyole und/oder Polyester-Polyole, wie sie zur Herstellung von zellförmigen und homogenen Polyurethanschaumstoffen an sich bekannt sind und die zum Beispiel in der DE-A 2 832 253 (Seiten 11-18) beschrieben werden.

Als Beispiele genannt seien hier:
(Standard) Polyether mit Hydroxylgruppen (Funktionalitäten) von vorzugsweise 2 und 3;
mit SAN (Styrol-Acryl -Nitril) Feststoff gefüllte (Standard) Polyether mit Hydroxylgruppen (Funktionalitäten) von vorzugsweise 2 und 3;
Hypersoft-Polyether mit Funktionalitäten von vorzugsweise 3;
reaktive Polyether mit primären Hydroxylgruppen vorzugsweise mit Funktionalitäten von 3, 5 und 6;
mit SAN (Styrol-Acryl -Nitril)-Feststoff bzw. PHD (Polyharnstoffdispersion) gefüllte reaktive Polyether mit primären Hydroxylgruppen vorzugsweise mit einer Funktionalität von 3;
mit TDI präpolymerisierte reaktive Polyole, vorzugsweise mit einer Funktionalität von 3;
mit TDI präpolymerisierte nicht reaktive Polyole auch als so genannte "Quasi-Präpolymere" (QPP) fachbekannt, vorzugsweise mit einer Funktionalität von 3, bei denen die Polyole gegenüber dem TDI im Überschuss vorliegen;
Polyole auf Basis nachwachsender Rohstoffe und unterschiedlicher Funktionalitäten. Naturölprodukte mit unterschiedlicher Anzahl von Hydroxylgruppen, wobei als nichtbeschränkendes Beispiel Rizinusöl zu nennen ist;
Verbindungen mit Aminogruppen oder Hydroxylgruppen, die als Kettenverlängerer oder Vernetzer dienen und in der Regel 2 bis 8, vorzugsweise 2 bis 4, gegenüber Isocyanaten reaktionsfähige Wasserstoffatome aufweisen, beispielsweise Diethanolamin, Triethanolamin, Diisopropanolamin, Sorbitol, Glycerin und Harnstoff.

Diese Weichschaumstoffe umfassen, ebenso wie die nachfolgend genannten Hartschaumstoffe, optional einen oder mehrere zusätzliche Substanzen, ausgewählt unter: Katalysatoren der an sich bekannten Art, wie tertiären Aminen und reaktiven (einbaubaren) Aminen; Zinn(II)-Verbindungen und Zink-Verbindungen, oberflächenaktiven Zusatzstoffen, wie Emulgatoren, Schaumstabilisatoren, Flammschutzmitteln, Sorbitol, Glycerin, Diaminen, Harnstoff, tertiären Aminen, und Stabilisatoren auf Siloxanbasis oder Nicht-Siloxanbasis.

Die Hartschaumstoffe dieser besonderen Weiterbildung sind beispielsweise zu finden in Produkten wie Dämmplatten (auch als Sandwich-Elemente mit verschiedenen Deckschichten), Ortschaumstoffen, Spritzschaumstoffen, mit dem Überschichtungsverfahren hergestellten Schaumstoffen, Schaumstoffen für Sonnenkollektorfüllungen, Schaumstoffen für Rohrisolierungen, Füll- und Montageschaumstoffen und Blockschaumstoffen. Die Zusammensetzungen sind dem Fachmann hinreichend bekannt und werden zum Beispiel in der Schrift EP 0 318 784 A2 umfassend beschrieben.

Vorzugsweise umfassen diese Hartschaumstoffe folgende Bestandteile oder bestehen aus Kombinationen zweier oder mehrerer Vertreter daraus:
Isocyanate:
2,4- und/oder 2,6-Toluylendiisocyanat (TDI) sowie beliebige Gemische dieser Isomere;
4,4'- und/oder 2,2'- Diphenylmethandiisocyanate (MDI) sowie beliebige Gemische dieser Isomere;
Polymer MDI ("Roh" - MDI) sowie mit mehrwertigen Polyolen (vorzugsweise zwei- und/oder dreiwertigen Polyethern) präpolymerisiertes MDI;
beliebige Gemische von TDI und MDI aus vorgenannten Isomeren und Ausbildungen, vorzugsweise jedoch Polymer MDI (Roh-MDI).

Neben Schaumstoffen sind weitere Beispiele für Polyurethan in dem Polyurethan enthaltenden Material Polyurethan-Elastomere und Polyurethan-Duroplaste.

Das erfindungsgemäße Verfahren kann diskontinuierlich, kontinuierlich oder semikontinuierlich durchgeführt werden.

Beim diskontinuierlichen Verfahren, auch als Batch-Verfahren bekannt, werden die Ausgangsstoffe in ein Reaktionsgefäß eingebracht, den Reaktionsbedingungen ausgesetzt, und anschließend das Reaktionsprodukt nach Ablauf der Behandlungsdauer entnommen. Anschließend wird das Reaktionsgefäß mit einer neuen Charge an Ausgangsstoffen beladen.

Beim kontinuierlichen Verfahren wird nicht lediglich einmalig vor Reaktionsbeginn Polyurethan enthaltendes Material sowie Harnstoff enthaltende wässrige Lösung in das Reaktionsgefäß eingespeist, sondern kontinuierlich. Analog wird ebenfalls kontinuierlich flüssiges Verfahrensmedium entnommen. Üblicherweise erfolgen die Einspeisung und die Entnahme an unterschiedlichen Stellen, insbesondere kann das Polyurethan enthaltende Material zusammen mit der Harnstoff enthaltenden wässrigen Lösung von der Stelle der Einspeisung bis zur Stelle der Entnahme geschoben oder durch aktive Fördermittel innerhalb des Reaktionsgefäßes transportiert werden. Entsprechend liegt an Stellen nahe der Einspeisungsstelle ein geringer Abbaugrad vor, wobei das Polyurethan enthaltene Material zusammen mit der Harnstoff enthaltenden wässrigen Lösung zur Entnahmestelle wandert oder transportiert wird und mit zunehmender Annäherung an die Entnahmestelle einen höheren Abbaugrad in Bezug auf das Polyurethan aufweist.

Semikontinuierliche Verfahren stellen jede mögliche Übergangsform zwischen den beiden vorstehend genannten Verfahren dar. Beispielsweise kann die Entnahme des flüssigen Verfahrensmediums nach Durchführung eines diskontinuierlichen Verfahrens nicht im Wesentlichen vollständig erfolgen. Als weiteres Beispiel kann als Abwandlung eines kontinuierlichen Verfahrens die Einspeisung von neuem Polyurethan enthaltenden Material und/oder Harnstoff enthaltender wässriger Lösung nicht ständig erfolgen, so dass nicht zu jedem Zeitpunkt, sondern vielmehr nur zu bestimmten Zeitpunkten, beispielsweise periodisch, oder nach Feststellung eines bestimmten Abbaugrads des im Reaktionsgefäß befindlichen Polyurethans neues Polyurethan enthaltendes Material und/oder Harnstoff enthaltende wässrige Lösung eingespeist wird.

Als Reaktionsgefäß kann beispielsweise jede Art von fachüblicher Vorrichtung verwendet oder vom Fachmann für die Zwecke des erfindungsgemäßen Verfahrens einfach angepasst werden. Beispiele hierfür sind Druckgefäße oder Druckreaktoren, die für eine chargenweise Befüllung ausgelegt sind und Reaktionsvolumina vom Labormaßstab, beispielsweise 0,1 bis 10 Liter über Zwischenbereiche von 10 Liter bis 1 Kubikmeter bis hin zum großindustriellen Maßstab im Bereich von 1 Kubikmeter bis zu Dutzenden oder Hunderten von Kubikmetern bereitstellen. Alternativ können die Druckgefäße oder Druckreaktoren für einen kontinuierlichen oder semikontinuierlichen Betrieb ausgelegt sein und Druckschleusen für das Einspeisen von Polyurethan enthaltendem Material beziehungsweise die Entnahme des flüssigen Verfahrensmediums umfassen.

Das Polyurethan enthaltende Material kann in unzerkleinerter Form eingesetzt werden. Beispielsweise kann ein Schaumstoff aus Polyurethan mit der Harnstoff enthaltenden wässrigen Lösung benetzt oder damit vollgesogen werden und anschließend auf die Temperatur im Bereich von 190°C bis 250°C erhitzt werden.

Vorzugsweise wird das Polyurethan enthaltende Material jedoch in zerkleinertem Zustand eingesetzt. Dabei können fachübliche Zerkleinerungsverfahren verwendet werden, beispielsweise kann das Polyurethan enthaltende Material zerschnitten, zerrissen, in Flocken geraspelt, geschreddert, granuliert, zermahlen oder pulverisiert werden, gegebenenfalls nach vorheriger Temperaturabsenkung zur Erhöhung der Sprödigkeit. Nichtlimitierende Beispiele für die Größe der dabei erhaltenen Zerkleinerungsprodukte sind etwa 0,5 cm³ bis 10 cm³ (0,5 ml bis 10 ml), wie etwa 1 cm³ bis 5 cm³, insbesondere für poröses oder eine große Oberfläche aufweisendes Polyurethan enthaltendes Material, oder Zerkleinerungsprodukte mit einem Durchmesser, gemessen an der größten Stelle, von maximal etwa 10, 5, 2, 1, 0,5, 0,1, 0,05 oder 0,01 Millimeter. Die Zerkleinerung kann während der Durchführung des Verfahrens durch entsprechende Vorrichtungen im Reaktionsgefäß erfolgen, vorzugsweise wird jedoch das Reaktionsgefäß mit Polyurethan enthaltendem Material in bereits zerkleinerter Form befüllt. Sofern das Polyurethan enthaltende Material nicht lediglich mit der Harnstoff enthaltenen Lösung benetzt ist, sondern vollständig darin aufgenommen ist, liegt im Wesentlichen eine Suspension des Polyurethan enthaltenden Materials in der Harnstoff enthaltenden wässrigen Lösung vor.

Bezogen auf die Gesamtmasse der wässrigen Lösung beträgt der Anteil des Harnstoffs in der wässrigen Lösung 1 bis 45 Massenprozent, insbesondere 1 bis 20 Massenprozent, beispielsweise 1 bis 10 Massenprozent, wie beispielsweise 1 bis 7 Massenprozent, beispielsweise 1,5 bis 5 Massenprozent, 1,5 bis 4 Massenprozent, 2 bis 4 Massenprozent, 2,5 bis 3,5 Massenprozent, oder 3 Massenprozent. Beispiele für weitere Bereiche oder weitere Konzentrationen sind 5 bis 10 Massenprozent, 1 Massenprozent, 5 Massenprozent, 7,5 Massenprozent und 10 Massenprozent. Bevorzugt unter dem Gesichtspunkt des Verhältnisses zwischen einzusetzender Menge an Harnstoff und erreichtem Abbaugrad von Polyurethan ist ein Bereich zwischen 1 Massenprozent und 10 Massenprozent, beispielsweise 2,5 bis 10 Massenprozent, 2 Massenprozent bis 7,5 Massenprozent, wie etwa 3 Massenprozent bis 5 Massenprozent.

Das Verfahren wird vorzugsweise durchgeführt unter Luftabschluss, wobei optional geringe Restmengen an Luft, beispielsweise 20% oder 10 % des Volumens des Reaktionsgefäßes, in welchem das Abbauverfahren durchgeführt wird, zulässig sind, oder wird unter Luftausschluss, oder in Gegenwart eines Inertgases, beispielsweise Stickstoffgas durchgeführt. Vorzugsweise wird das Verfahren in Gegenwart eines Inertgases durchgeführt.

In einer besonderen Ausführungsform umfasst die wässrige Lösung 2,5 bis 10 Massenprozent Harnstoff, und das Erhitzen erfolgt über einen Zeitraum von 45 bis 250 Minuten, insbesondere über einen Zeitraum von 45 bis 240 Minuten.

Gemäß einer Ausführungsform beträgt das Verhältnis zwischen Harnstoff enthaltender wässriger Lösung und Polyurethan enthaltendem Material 0,2 ml/g bis 5 ml/g, wie etwa 0,4 ml/g bis 5 ml/g, beispielsweise 0,2 ml/g bis 2,5 ml/g, 0,2 ml/g bis 2,0 ml/g, 0,2 ml/g bis 1,7 ml/g, 0,2 ml/g bis 1,2 ml/g oder 0,2 ml/g bis 1,0 ml/g, oder beispielsweise 0,4 ml/g bis 2,5 ml/g, 0,4 ml/g bis 2,0 ml/g, 0,4 ml/g bis 1,7 ml/g, 0,4 ml/g bis 1,2 ml/g oder 0,4 ml/g bis 1,0 ml/g. Insbesondere bei einem geringen Verhältnis zwischen eingesetztem Volumen der Harnstoff enthaltenden wässrigen Lösung und der damit behandelten Menge an Polyurethan ergibt sich eine effiziente Nutzung des Volumens, welches für die Durchführung des Abbauverfahrens zur Verfügung steht. Bezogen auf einen Einsatz mit Polyurethan-Schaum stellt eine Menge von 0,2 ml/, insbesondere 0,25 ml/g, insbesondere 0,29 ml/g, und insbesondere 0,4 ml/g eine Untergrenze dar, bei welcher der Polyurethan-Schaum noch ausreichend mit der wässrigen harnstoffhaltigen Lösung befeuchtet ist, um den gewünschten Abbau von Polyurethan zu ermöglichen.

In dem Verfahren ist optional als weiterer Verfahrensschritt vorgesehen, das nach dem Erhitzen erhaltene flüssige Verfahrensmedium wenigstens teilweise zu gewinnen. Dementsprechend wird das flüssige Verfahrensmedium ganz oder teilweise aus dem Reaktionsgefäß entnommen. Es steht damit für sich optional anschließend weitere Schritte zur Verfügung, beispielsweise für eine Fraktionierung in einzelne Substanzen oder Substanzgruppen, die gezielter entsorgt werden können oder im Optimalfall als Rohstoffquellen zur Verfügung stehen.

Gemäß einer Ausführungsform ist vorgesehen, dass aus dem gewonnenen flüssigen Verfahrensmedium darin enthaltene Feststoffe entfernt werden, sofern solche darin enthalten sind. Dabei kann es sich beispielsweise um Metallteile, Partikel anderer Kunststoffe, oder partikuläre Reaktions- oder Abbauprodukte der Polyurethane oder Polyurethan-Bestandteile handeln. Verfahren zur Entfernung von Feststoffen sind dem Fachmann bekannt, und umfassen beispielsweise Sedimentation, Zentrifugation oder Filtration.

Die 1 bis 45 Massenprozent Harnstoff enthaltende wässrige Lösung ist gemäß einer bevorzugten Ausführungsform frei von Polyol, beispielsweise frei von Diol, und/oder frei von Carbonsäuren und/oder frei von Ammoniak. Dadurch werden vorteilhaft umweltbelastende Substanzen vermieden. Gemäß einer besonderen Ausführungsform besteht die wässrige Lösung aus Wasser und 1 bis 45 Massenprozent Harnstoff.

Bei der Durchführung des Abbauverfahrens erfolgt eine partielle oder vollständige Überführung des Polyurethans in das flüssige Verfahrensmedium. Dementsprechend lassen sich ursprünglich in fester Form vorliegende, Polyurethan enthaltende Materialien, die insbesondere für den Fall, dass das Polyurethan in Form eines Schaumstoffs vorliegt, einen hohen Volumenbedarf haben, in leichter handhabbare Flüssigkeiten, nämlich das flüssige Verfahrensmedium, mit geringerem Volumenbedarf überführen. Weiterhin besteht die Möglichkeit, die im flüssigen Verfahrensmedium enthaltenen Substanzen zu fraktionieren und/oder zu isolieren und/oder gegebenenfalls einer Weiterverwendung oder Wiederverwendung zuzuführen.

Die vorliegende Erfindung betrifft dementsprechend ein Verfahrensmedium, erhältlich oder erhalten durch ein hierin beschriebenes Verfahren, , wobei das Erhitzen bei einer Temperatur von 190°C bis 250°C über einen Zeitraum von 20 Minuten bis 240 Minuten erfolgt, wobei die wässrige Lösung 1 bis 10 Massenprozent Harnstoff enthält und das Verhältnis zwischen Harnstoff enthaltender wässriger Lösung und Polyurethan enthaltendem Material 0,4 ml/g bis 5 ml/g beträgt. Sie betrifft insbesondere ein derartiges Verfahrensmedium, welches Polyole enthält, die zur Herstellung des mittels des Verfahrens abgebauten Polyurethans verwendet wurden, oder Modifizierungen von derartigen Polyolen enthält, und/oder Diamine oder Modifizierungen von derartigen Diaminen enthält, wobei die Diamine Vorstufen der Di-Isocyanate darstellen, die zur Herstellung des mittels des Verfahrens abgebauten Polyurethans verwendet wurden. Nicht-beschränkende Beispiele für Modifizierungen sind Ringbildungen oder Deaminierungen. Ein derartiges Verfahrensmedium enthält somit wertvolle Rohstoffe für die chemische Industrie, insbesondere für die Polyurethan-Kreislaufwirtschaft, die sich - gegebenenfalls nach Isolierung aus dem Verfahrensmedium - erneut verwenden lassen.

Weitere Vorteile, Merkmale und Einzelheiten ergeben sich aus der nachfolgenden Beschreibung, in der - gegebenenfalls unter Bezug auf die Figuren - zumindest ein Ausführungsbeispiel im Einzelnen beschrieben ist.
Figur 1 zeigt ein dreidimensionales Diagramm zur Darstellung des Abbaugrads von Polyurethan enthaltendem Material in Abhängigkeit von der Temperatur und der Dauer der Verfahrensdurchführung.
Figur 2 zeigt ein dreidimensionales Diagramm zur Darstellung des Einflusses von Harnstoff auf den Abbau verschiedener Polyurethan enthaltender Materialien bei einer gewählten Erhitzungsdauer.
Figur 3 zeigt ein dreidimensionales Diagramm zur Darstellung des Einflusses von Harnstoff auf den Abbau eines Polyurethan enthaltenden Materials in einer Zeitreihe.

### Beispiel 1 - Polyurethan-Weichschaumstoffe

Zwei Polyurethan-Weichschaumstoffe auf TDI-Basis bzw. MDI-Basis (Produktbezeichnungen: R 4030 bzw. R 5535, Quelle: Eurofoam Deutschland GmbH Schaumstoffe, Wiesbaden, Deutschland) wurden mit 20,0 g beziehungsweise 19,6 g in 32,2 ml beziehungsweise 35,7 ml 7,5-%iger Harnstofflösung aufgenommen und 180 Minuten bei 190 °C in einem geschlossenen Druckgefäß behandelt.

**Tabelle 1:**

| Einwaage von Polyurethan-Weichschaumstoff [g] | Harnstoffgehalt der wässrigen Lösung [Massen-%] | eingesetztes Volumen der wässrigen Lösung [ml] | Abbau [%] |
|---|---|---|---|
| 20,0 (R 4030) | 7,5 | 32,2 | 50 |
| 19,6 (R 5535) | 7,5 | 35,7 | 90 |
| | | | |
| 180 min bei 190°C | | | |

Die entnommenen Proben wurden nach Abkühlung mittels visueller Begutachtung semi-quantitativ hinsichtlich des Abbaugrads von festem Polyurethan und dementsprechend Verschwinden von feststellbarem Festmaterial und Überführung in das flüssige Verfahrensmedium eingeordnet, wobei (wie in allen anderen nachfolgenden Versuchen) "0 %" dafür stehen, dass noch kein Abbau von Polyurethan stattfand, und dementsprechend die ursprünglich eingesetzte Feststoffmenge von Polyurethan noch vollständig vorlag, wohingegen "100%" für einen vollständigen Abbau und entsprechende Überführung des Polyurethans in das flüssige Verfahrensmedium stehen.

In beiden Fällen erfolgte ein partieller Abbau, wobei noch unterschiedliche Mengen an Feststoffen in Form offenbar verdichteter Schaumstoffrückstände im flüssigen Verfahrensmedium vorlagen.

### Beispiel 2 - Polyurethan-Integralschaum

Zwei Proben von flexiblem Polyurethan-Integralschaum auf Etherbasis beziehungsweise ein unter der Marke Colo-Fast^{®} (BASF, SE, Ludwigshafen, Deutschland) vertriebenes Gieß-Elastomer wurden mit Einwaagen von 25 g bzw. 35 g in 40 ml 3%-iger Harnstofflösung aufgenommen und 240 Minuten bei 245 °C in einem geschlossenen Druckgefäß behandelt, das zu 80 % gefüllt war.

**Tabelle 2:**

| Einwaage von Polyurethan-Integralschaum [g] | Harnstoffgehalt der wässrigen Lösung [Massenprozent] | eingesetztes Volumen der wässrigen Lösung [ml] | Abbau [%] |
|---|---|---|---|
| 25 (Etherbasis) | 3 | 40 | 100 |
| 35 (Colo-Fast) | 3 | 40 | 100 |
| | | | |
| 240 min bei 245°C | | | |

Es erfolgte ein vollständiger Abbau der eingesetzten Polyurethan-Materialien, jedoch wurde ein geringer Anteil an Partikeln festgestellt, die möglicherweise auf eine Polymerisierung zurückzuführen waren.

### Beispiel 3 - nicht-geschäumtes Polyurethan

In einer Versuchsreihe wurden feste, nicht geschäumte Polyurethane für das Abbauverfahren verwendet. Dabei wurden aus Polyurethan bestehende blaue Kunststoffschläuche des Typs "PUN" (Festo Gesellschaft m.b.H, Wien, Österreich) zerkleinert und jeweils 1 g in 25 ml einer wässrigen Harnstofflösung mit einem Harnstoffgehalt von 1 Massenprozent, 5 Massenprozent, 7,5 Massenprozent bzw. 10 Massenprozent über einen Zeitraum von 150 Minuten auf 210 °C in einem geschlossenen Druckgefäß erhitzt. Die Versuchsreihe diente im Wesentlichen der Bestimmung des Einflusses der Harnstoffkonzentration und der Ermittlung der Abbaubarkeit von festen Polyurethanen

**Tabelle 3:**

| Einwaage von Polyurethan-Schlauch [g] | Harnstoffgehalt der wässrigen Lösung [Massenprozent] | eingesetztes Volumen der wässrigen Lösung [ml] | Abbau [%] |
|---|---|---|---|
| 30 | 1 | 25 | 100 |
| 30 | 5 | 25 | 100 |
| 5 | 7,5 | 20 | 100 |
| 5 | 7,5 | 20 | 100 |
| 30 | 7,5 | 25 | 100 |
| 30 | 10 | 25 | 100 |
| | | | |
| 150 min bei 210°C | | | |

Als Ergebnis wurde festgestellt, dass in allen Ansätzen im erhaltenen flüssigen Verfahrensmedium keine Anteile des ursprünglich eingesetzten festen Polyurethans mehr feststellbar waren. Somit sind unter den genannten Versuchsbedingungen nicht-geschäumte Polyurethane abbaubar.

### Beispiel 4 - duroplastisches Polyurethan

Als weiteres Beispiel für nicht-geschäumte Polyurethane wurde ein Duroplast-Polyurethan-Kern mit einem Raumgewicht von 165 g/l, der in Skis zum Einsatz kommt, für das Abbauverfahren verwendet. Der Kern wurde zerkleinert und 15 g in 40 ml einer wässrigen Harnstofflösung mit einem Harnstoffgehalt von 3 Massenprozent über einen Zeitraum von 150 Minuten auf 210 °C in einem geschlossenen Druckgefäß mit einem Füllungsgrad von 80 % des verfügbaren Volumens (restliches Volumen 20 %: Luft) erhitzt.

**Tabelle 4:**

| Einwaage von Polyurethan-Ski-Kernmaterial [g] | Harnstoffgehalt der wässrigen Lösung [Massenprozent] | Volumen der wässrigen Lösung [ml] | Abbau [%] |
|---|---|---|---|
| 15 | 3 | 40 | 100 |
| | | | |
| 150 min bei 210°C | | | |

Als Ergebnis wurde festgestellt, dass in der erhaltenen flüssigen Phase keine Anteile des ursprünglich eingesetzten nicht-geschäumten Polyurethans mehr feststellbar waren.

### Beispiel 5 - elastomere Polyurethane

Als Beispiele für elastomere Polyurethane wurden Federn aus mikrozellärem Polyurethan, die unter der Marke Cellasto^{®} vertrieben werden (BASF, SE, Ludwigshafen, Deutschland), sowie das Gieß-Elastomer Colo-Fast^{®} (BASF SE, Ludwigshafen, Deutschland) verwendet. Die Proben wurden zerkleinert und in getrennten Ansätzen in 40 ml einer wässrigen Harnstofflösung mit einem Harnstoffgehalt von 3 Massenprozent über einen Zeitraum von 150 Minuten auf 210 °C in einem geschlossenen Druckgefäß mit einem Füllungsgrad von 80 % des verfügbaren Volumens (restliches Volumen 20 %: Luft) erhitzt.

**Tabelle 5:**

| Einwaage von elastomerem Polyurethan [g] | Harnstoffgehalt der wässrigen Lösung [Massenprozent] | eingesetztes Volumen der wässrigen Lösung [ml] | Abbau [%] |
|---|---|---|---|
| 35 (Cellasto^{®} (Probe 1) | 3 | 40 | 100 |
| 40 (Cellasto^{®} (Probe 2) | 3 | 40 | 100 |
| 35 (Colo-Fast^{®}) | 3 | 40 | 100 |
| | | | |
| 150 min bei 210 °C | | | |

Als Ergebnis wurde festgestellt, dass in allen Ansätzen in der erhaltenen flüssigen Phase keine Anteile des ursprünglich eingesetzten elastomeren Polyurethans mehr feststellbar waren.

### Beispiel 6 - Zeitreihe

In einer Versuchsreihe wurden in einer wässrigen Harnstofflösung mit einem Harnstoffgehalt von 7,5 Massenprozent die bereits erwähnten Polyurethan-Schaumstoffe R 4030 und R 5535 bei der Temperatur von 230 °C in einem geschlossenen Druckgefäß für 60 Minuten, 90 Minuten oder 120 Minuten erhitzt.

**Tabelle 6:**

| Einwaage von Polyurethan-Schaumstoff [g] | Harnstoffgehalt der wässrigen Lösung [Massenprozent] | eingesetztes Volumen der wässrigen Lösung [ml] | Dauer [min] | Abbau [%] |
|---|---|---|---|---|
| 9,98 (R 4030) | 7,5 | 25 | 60 | 50 |
| 10,47 (R 5535) | 7,5 | 25 | 60 | 50 |
| 9,93 (R 4030) | 7,5 | 25 | 60 | 50 |
| 10,36 (R 5535) | 7,5 | 25 | 60 | 50 |
| 10,08 (R 4030) | 7,5 | 25 | 90 | 90 |
| 10,04 (R 5535) | 7,5 | 25 | 90 | 90 |
| 10,15 (R 4030) | 7,5 | 25 | 120 | 100 |
| 10,23 (R 5535) | 7,5 | 25 | 120 | 100 |
| | | | | |
| verschiedene Zeitdauern bei 230°C | | | | |

Bei der als 50 % eingestuften Abbaurate gingen die Schaumstoffstrukturen in eine Auflösung über und führten zu einer pastösen Konsistenz, bei der als 90 % eingestuften Abbaurate war die Schaumstoffstruktur fast vollständig verflüssigt, unter 100-prozentigem Abbau zu 100 % verflüssigt.

### Beispiel 7 - Zeitreihe und Temperaturreihe

Nachdem die vorangegangenen Versuche ergeben hatten, dass prinzipiell jede Art von Polyurethan enthaltendem Material mittels des Verfahrens abgebaut werden kann, wurden in einer neuen Versuchsreihe mit dem Ziel der systematischen Ermittlung einer Temperaturabhängigkeit und Zeitabhängigkeit des Abbaus eine Mischung aus verschiedenen Polyurethan enthaltenden Materialien eingesetzt, nämlich eine Mischung aus gleichen Gewichtsanteilen bei einer Einwaage von jeweils 4,15 g
a) Standardschaum (standard foam grade TDI 80-based) mit Füllstoff (Calciumcarbonat) und SAN-Polymer-Partikeln (N 4045 WS),
b) stark rückfederndem Polyurethanschaumstoff [HR (high resilience) foam grade TDI 80/TDI65-based) mit Füllstoff (Calciumcarbonat) und SAN-Polymer-Partikeln (R 4040 WS),
c) stark rückfederndem Polyurethanschaumstoff [HR (high resilience) foam grade MDI-based) mit Füllstoff (Calciumcarbonat) und SAN-Polymer-Partikeln (R 5535 WS), und
d) viskoelastischem Schaum (viscoelastic foam grade MDI-based - V5018 WS, Eurofoam Deutschland GmbH Schaumstoffe) ohne Füllstoffe.

Das dermaßen erhaltene Polyurethan enthaltende Material wurde in geschredderter Form mit 3%-iger Harnstofflösung in einem Verhältnis von 0,582 ml Harnstofflösung pro Gramm Polyurethan enthaltendem Material versetzt. Unter Durchführung von Temperaturreihen wurden Proben in abgeschlossenen Druckgefäßen unter Einstellung eines Gleichgewichtsdrucks auf 190 °C, 210°C, 230 °C bzw. 245 °C in einem Wärmeschrank erhitzt und nach einer Verweildauer von 60 Minuten, 90 Minuten, 120 Minuten, 180 Minuten beziehungsweise 240 Minuten bei der jeweiligen Temperatur entnommen. Für jeden Zeitpunkt wurden parallel drei Proben angesetzt und ausgewertet.

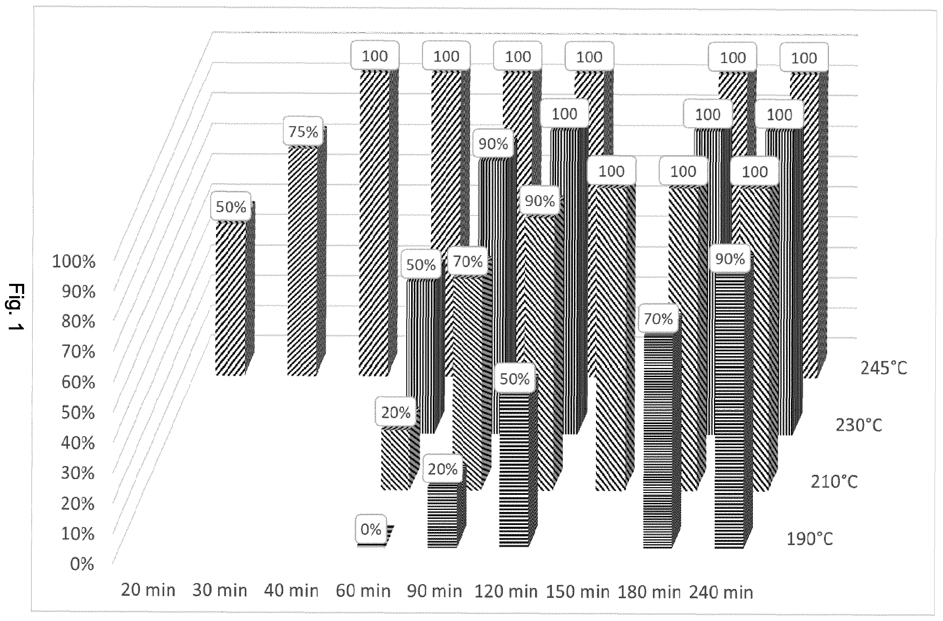

Die entnommenen Proben wurden nach Abkühlung mittels visueller Begutachtung unter Bildung des Mittelwerts jeweiligen drei Proben semi-quantitativ hinsichtlich des Abbaugrads von festem Polyurethan und dementsprechend Verschwinden von feststellbarem Festmaterial und Überführung in das flüssige Verfahrensmedium eingeordnet. Das Ergebnis ist in Figur 1 dargestellt, wobei auf der waagrechten x-Achse die Dauer der Temperaturbehandlung bei einer gegebenen Temperatur in Minuten angegeben ist, auf der senkrechten z-Achse der semi-quantitative Abbaugrad in Prozent angegeben ist, und auf der in die Bildebene führenden y-Achse die drei gewählten Temperaturen von 190 °C, 210 °C, 230 °C und 245 °C aufgetragen sind. Es zeigt sich, dass mit zunehmender Temperatur bis 245° schneller höhere Abbauraten erzielbar sind.

### Beispiel 8 - Kontrollversuch ohne Überdruck

In einem Kontrollversuch wurden in zwei getrennten Ansätzen jeweils 2 g TDI-Polyurethan-Schaumstoff (Produktbezeichnung: R 4030; Quelle: Eurofoam Deutschland GmbH Schaumstoffe, Wiesbaden, Deutschland) bzw. MDI- Polyurethan-Schaumstoff (Produktbezeichnung: R5535; Quelle: Eurofoam Deutschland GmbH Schaumstoffe, Wiesbaden, Deutschland) mit jeweils 40 ml 7,5%iger Harnstofflösung versetzt und in einem Glaskolben einer Temperatur von 100 °C bis 110 °C bei Umgebungsdruck in einem Wärmeschrank behandelt, wobei der Flüssigkeitsverlust durch Zugabe von Wasser ergänzt wurde. Nach 4,5 Stunden konnte keinerlei Abbau festgestellt werden.

### Beispiel 9 - erster Kontrollversuch ohne Harnstoff bei Überdruck

Als weiterer Kontrollversuch wurden vier unterschiedliche Ansätze, nämlich
16,6 g schwarzer PUR-Integralschaum (Fenster-Dämmmaterial) in 9,66 ml H₂0,
16,6 g weißer PUR-Integralschaum (Komponente eines Schuhs) in 9,66 ml H₂0,
14,0 g PUR-Halbhartschaum (BASF) in 8,15 ml H₂0, und
16,0 g PUR-Elastomer (Cellasto^{®} von BASF) in 9,66 ml H₂0
jeweils für 30 min bei 245 °C in abgeschlossenen Druckgefäßen erhitzt, wobei die
angegebenen Wassermengen entweder frei von Harnstoff waren oder 3 Massenprozent Harnstoff enthielten.

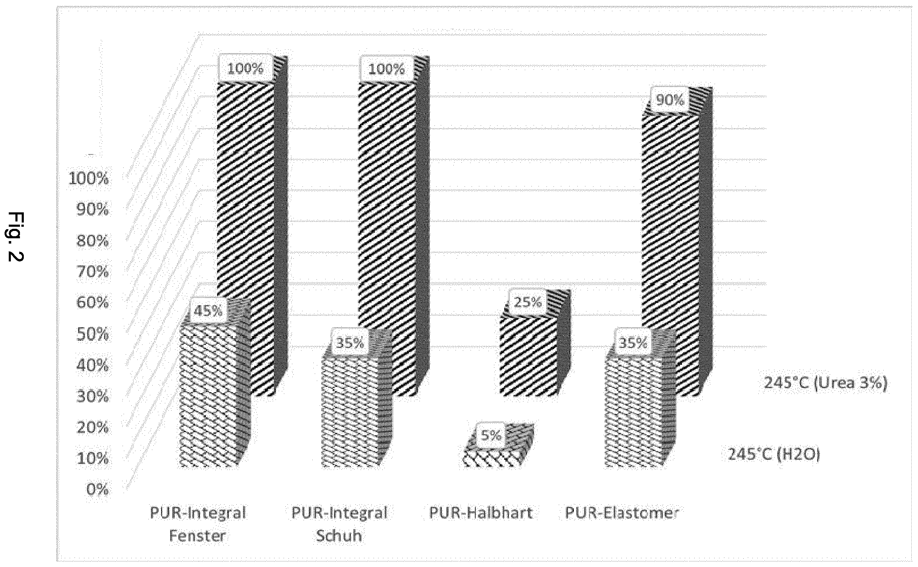

Die Ergebnisse sind in Figur 2 dargestellt, wobei die vorstehend aufgelisteten Ansätze als "PUR-Integral Fenster", "PUR-Integral Schuh", "PUR-Halbhart" bzw. "PUR-Elastomere" bezeichnet sind. Auf der y-Achse ist der Grad der Umsetzung in Prozent angegeben. Es zeigte sich, dass in allen Fällen durch Zugabe von Harnstoff eine deutliche Erhöhung des Abbaus zu erzielen war. Im Falle der Integralschäume und des Elastomers konnte dadurch ein vollständiger bzw. fast vollständiger Abbau des eingesetzten Festmaterials ausgelöst werden, und auch im Falle des PUR-Halbhart-Schaums, der bei einer Erhitzungsdauer von 30 Minuten weniger vollständig abgebaut wurde, war dennoch eine deutliche Steigerung der Abbaurate bei Zugabe von Harnstoff zu verzeichnen. Der verschlechterte Abbau des Halbhart-Schaumes könnte durch eine verschlechterte Wärmeübertragung in dem Schaumaterial bedingt sein, da der verwendete Halbhart-Schaum mit einem Raumgewicht von 133 g/L eine deutlich geringere Dichte hat als "PUR-Integral Fenster" (850 g/L), "PUR-Integral Schuh" (790 - 830 g/L) und "PUR-Elastomer" (400 - 550 g/L) aufweist. Insofern füllte der PUR-Halbhart-Schaum das Druckgefäß viel vollständiger aus, war dadurch schlechter mit der harnstofffreien oder harnstoffhaltigen wässrigen Lösung benetzbar und erschwerte aufgrund des höheren Luftvolumens in den Poren den Wärmetransfer.

### Beispiel 10 - zweiter Kontrollversuch ohne Harnstoff bei Überdruck

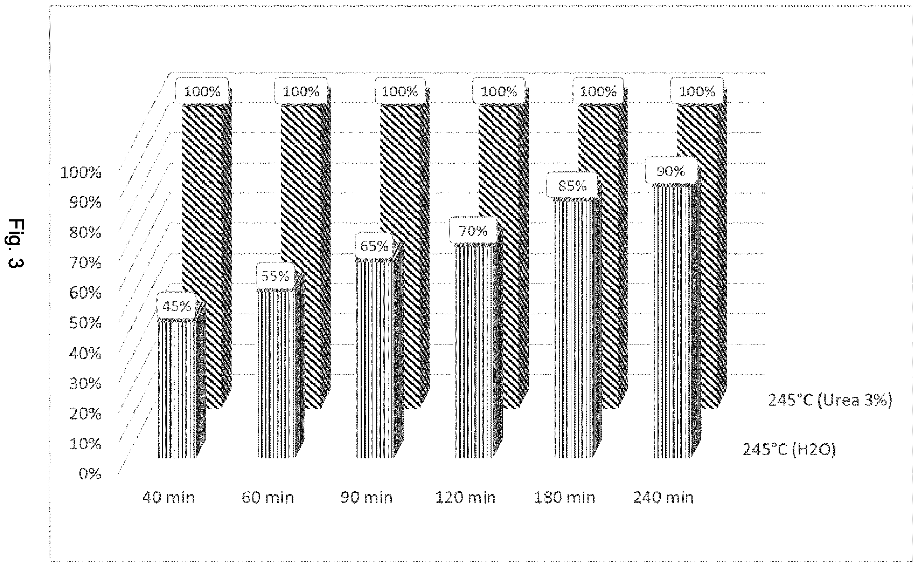

In einem weiteren Kontrollversuch wurden mehrfache Ansätze einer Mischung aus Polyurethan-Weichschaumstoffen (entweder 16,6 g in 9,66 ml Wasser oder 16,6 g in 9,66 ml Wasser, welches 3 Massenprozent Harnstoff enthielt) bei 245 °C in abgeschlossenen Druckgefäßen erhitzt und zu unterschiedlichen Zeitpunkten (40, 60, 90,120, 180 bzw. 240 min) entnommen. Im Vergleich mit den im Beispiel 9 verwendeten Proben entsprach die Mischung aus Polyurethan-Weichschaumstoffen hinsichtlich ihrer Materialeigenschaften am ehesten dem PUR-Halbhart-Schaum. Die Ergebnisse sind in Figur 3 dargestellt, wobei auf der x-Achse der Zeitpunkt in Minuten und auf der y-Achse der Grad der Umsetzung in Prozent angegeben ist. Es zeigte sich, dass in Gegenwart von 3 Massenprozent Harnstoff bereits ab einer Erhitzungsdauer von 40 Minuten ein vollständiger Abbau zu verzeichnen war, wohingegen in Harnstoff-freiem Wasser bei 40 Minuten lediglich ein teilweiser Abbau vorlag, der mit zunehmender Erhitzungsdauer zwar ebenfalls zunahm, jedoch in jedem Fall unter dem durch Behandlung mit Harnstoff-haltigen Wasser erzielbaren Abbau lag.

### Beispiel 11 - Versuch mit einfach aufgebautem Weichschaumsystem (single polyol foam), Analytik der Abbauprodukte

Zur Bestimmung der entstehenden Abbauprodukte nach Anwendung des Verfahrens wurde ein Polyurethan-Weichschaum mit möglichst einfachem chemischem Aufbau im Labor hergestellt (Tabelle 7). Dabei wurde zur Begrenzung der Einflussgrößen nur ein Standard-Ether-Polyol mit einem Molekulargewicht 3500 MW verwendet und als Isocyanat wurde Toluol-Diisocyanat TDI 80 eingesetzt. Das Raumgewicht des Schaumstoffs betrug 22 kg/m³

**Tabelle 7:**

| Rohstoff | Gewichtsteile |
|---|---|
| Polyol Standard Ether (3500 MW) | 100 |
| Amin BDE - Dabco BL 11 (Evonik Industries,Essen, Deutschland | 0,1 |
| Amin TEDA - Dabco 33 LV Evonik Industries, Essen, Deutschland) | 0,22 |
| Silikon Niax L 620 (Momentive Performance Materials, D-Leverkusen | 0,48 |
| Wasser dosiert | 4,48 |
| Zinnoctoat rein | 0,15 |
| TDI 80 | 50,81 |

Dieser PUR-Weichschaumstoff wurde zerkleinert und in einem 11,5 Liter-Büchi-Reaktor (Büchi AG, Uster, Schweiz) mit dem Verfahren behandelt. Dabei wurden 400 Gramm Schaumstoff mit 236 Milliliter einer 3%igen Wasser-Harnstofflösung vorbefeuchtet und in ein Edelstahl-Aufschluss-Gefäß (auch als Inliner-Gefäß bezeichnet) mit einem Volumen von ca. 10,2 Liter eingefüllt. Der Boden des Gefäßes wurde mit Aluminiumfolie mit einer Randhöhe von 5 cm ausgekleidet, um das entstehende flüssige Verfahrensmedium aufzufangen. Unter das in den Reaktor eingesetzte Inliner-Gefäß wurden 200 ml Wasser zur schnellen Erzeugung von Wasserdampf gegeben. Das erfindungsgemäße Verfahren wurde über einen Zeitraum von 240 min durchgeführt, wobei die Reaktor-Manteltemperatur zunächst 60 min auf 260°C gesetzt war, und anschließend 180 min auf 250°C. Der Reaktorinnenraum erreichte dabei am Ende eine Temperatur von knapp 230°C (229,3°C).

Um das erhaltene Verfahrensmedium chemisch zu analysieren, wurden 1 ml davon in 99 ml Acetonitril gelöst und 24 h auf einer Labor-Rüttelplatte gemischt. Anschließend wurde das Acrylonitril-Proben-Gemisch mit einem Spritzenvorsatzfilter (z.B. Chromafil Xtra, RC, 25 mm, 0,45 µm) gefiltert, bevor 1,5 Mikroliter der Probe direkt in ein Gaschromatographie-System vom Typ Agilent 8890 GC (Agilent, Santa Clara, USA) eingebracht. Die GC Säule wurde initial bei 40°C 2 Minuten gehalten und dann mit 10 Kelvin pro Minute von 40°C auf 250°C erwärmt. Am Ende schloss sich eine Haltezeit von 5 Minuten an. Insgesamt betrug die Mess-Zeit 28 Minuten. Die Messung erfolgte mit einem Split 1:10. Der Name der Methode lautet Ramp40-250_28min_1.5ml_split1-10.M. Das Spektrum, das mit dem gekoppelten Agilent 5977B GC/MSD Massenspektrometer System gemessen wurde, zeigt zwei ausgeprägte Peaks bei den Retentionszeiten 1.37 bis 1.75 Minuten und 13.71 Minuten. Die Auswertung der Spektren erfolgte über das Software-Paket "NIST17", das im Lieferumfang des im Beispiel 12 näher beschriebenen TG/STA-GC-MS-Systems enthalten war (National Institute of Standards and Technology, Gaithersburg, USA), weiterhin die Software "AMDIS32" für qualitative GC-MS-Analysen, die Datenbanken ALKANES, NISTCW, NISTDRUG, NISTEPA, NISTFAD, NISTFF, NISTTOX und PESTPLUS, sowie die Software MSSEARCH umfasste, wobei sich mit letztgenannter Software gezielt einzelne gemessene Scans aus mit den verwendeten Datenbanken abgleichen lässt. Die Auswertung der Peaks ist in Tabelle 8 dargestellt, wobei MF (Match Factor) der Abgleich des gemessen Spektrums zu den Spektren in den Datenbanken bedeutet, und RFM (Reverse Match Factor) der Abgleich der verfügbaren Datenbankspektren zu dem gemessenen Spektrum. In beiden Fällen wird ein spezieller Algorithmus verwendet, der in der Software hinterlegt ist. Diese Faktoren sind in der NIST-Software auf 1000 = 100% Übereinstimmung normiert.

**Tabelle 8:**

| Retentionszeit [min] | Substanz | MF | RMF | |
|---|---|---|---|---|
| 1.37 | Acetonitrile (CAS 75-05-8) | 897 | 900 | |
| 1.75 | Acetonitrile (CAS75-05-8) | 912 | 914 | |
| 13.71 | 1,3-Benzenediamine, 4-methyl-(CAS 95-80-7) | 962 | 963 | |

Der Acetonitril-Peak ist auf das verwendete Lösungsmittel Acetonitril zurückzuführen. Ein Synonym für das 1,3-Benzenediamine, 4-methyl- (CAS 95-80-7) ist 2,4-Diaminotolul. Der 2,4-Diaminotoluol-Peak weist darauf hin, dass diese Verbindung im Rahmen des erfindungsgemäßen Verfahrens erhalten werden kann. 2,4-Diaminotoluol ist eine Vorstufe der Toluol-Diisocyanats TDI 80, in welches es über Phosgenierung überführt werden kann, und stellt damit einen industriell wertvollen, wiederverwendbaren Rohstoff, insbesondere im Rahmen der Polyurethan-Wirtschaft dar.

Von dem Verfahrensmedium wurde ebenfalls eine GPC-Analyse (Gel-Permeations-Chromatographie) zu Bestimmung des Molekulargewichtes vorgenommen (externe Messung bei BASF Lemförde - Central Analytic Lemförde, BASF Polyurethanes GmbH, Elastogranstr 60, 49448 Lemfoerde, Germany).

Dabei wurde eine molare Massenverteilung mit einem deutlichen Hauptpeak bei 3700 g/mol gemessen. Die Viskosität des Verfahrensmediums wurde ebenfalls bestimmt. Sie liegt bei 1100 mPas. Ebenso wurde die OH-Zahl des Verfahrensmediums bestimmt - sie betrug 325 mg KOH/g.

Die Resultate unterstützen die Annahme, dass ein wesentlicher Bestandteil des Verfahrensmediums von Polyol gestellt wird. Es ist dem ursprünglich eingesetzten Polyol sehr ähnlich in der molaren Masse und hat (möglicherweise aufgrund in dem Verfahren erfahrenen Behandlung) eine leichthöhere Viskosität (Polyol Standard Ether -3500 MW = 700 - 900 mPa•s, OH 48) und eine höhere OH-Zahl.

Dementsprechend lassen sich mit dem erfindungsgemäßen Verfahren Polyole gewinnen und als wertvolle Rohstoffe einer Wiederverwendung zuführen.

### Beispiel 12 - Versuch mit PUR Weichschaumstoffmischung, Analytik der Abbauprodukte über TG - GC/MS

In diesem Beispiel wurde versucht, im Rahmen des erfindungsgemäßen Verfahrens entstehende Polyole im erhaltenen Verfahrensmedium nachzuweisen. Zu diesem Zweck sollte das Verfahrensmedium bei Temperaturen von etwa 380 °C thermisch zersetzt werden und aus den entstandenen Zersetzungsprodukten auf das Vorliegen von Polyolen im Verfahrensmedium geschlossen werden. Hierzu wurde der Weg über gekoppelte TG/STA-GC-MS Messungen gewählt (Kopplung von Gaschromatografie-Massenspektrometer mit thermischer Analyse, wobei TG für Thermogravimetrie und STA für "simultaneous thermal analyis" steht). Die verwendeten Messgeräte waren eine STA 449 F3 Jupiter (Netzsch, Selb, Deutschland) die über eine beheizte Transferline mit dem Gaschromatographie-System vom Typ Agilent 8890 GC (Agilent, Santa Clara, USA) und dem Massenspektrometer System Agilent 5977B GC/MSD verbunden war.

Zunächst wurde in einem vorbereitenden Schritt folgendermaßen eine Referenzdatenbank angelegt.

Dafür wurden die in der nachfolgenden Tabelle 9 aufgelisteten Polyole verwendet, da diese konstitutionelle Bestandteile der verwendeten Weichschaumstoffe in der später anhand der Referenzdatenbank tatsächlich untersuchten Mischung (vgl. Tabelle 10) darstellen.

**Tabelle 9:**

| Polyol | Zusatzbezeichnung in der AMDIS-Datenbank |
|---|---|
| Standard 3500 MW | STD_POLYOL |
| Standard 45% SAN Polyol | SAN_45% |
| HR 6000 MW Polyol | HR_6000 |
| HR 25% SAN Polyol | HR_SAN |
| HR high functional Polyol | HR_HF |
| Hypersoft Polyol | HYPERSOFT |
| Viscoelastic Polyol | VE8420 |

Diese wurden jeweils einzeln bei 380°C in der thermischen Abbaustufe des vorstehend genannten TG/STA-GC-MS-Systems zersetzt. Die Hauptpeaks wurden über die Datenbanken von NIST und AMDIS (vgl. Beispiel 11) bestimmt, und der Name der zugeordneten Verbindung laut diesen Datenbanken und die GC-Retentionszeiten der Zersetzungsprodukte in der Referenzdatenbank hinterlegt, wobei jedem Zersetzungsprodukt die jeweilige Zusatzbezeichnung laut Tabelle 9 hinzugefügt wurde. Traten die gleichen Zersetzungsprodukte bei verschiedenen Polyolen auf, wurde die jeweilige Zusatzbezeichnung ergänzt. Diese Zersetzungsprodukte mit dem entsprechenden identifizierenden Produktkürzel finden sich dann in Tabelle 11 wieder (wobei in Tabelle 11 die Zersetzungsprodukte der Mischung gemäß Tabelle 10 dargestellt sind).

Nach Erstellung der Referenzdatenbank wurde zur Klärung der Frage, inwieweit nach Durchführung des erfindungsgemäßen Verfahrens Polyole im Verfahrensmedium auftreten, eine Mischung aus Weichschaumstoffen dem erfindungsgemäßen Verfahren unterworfen.

Hierzu wurden 400 Gramm einer Mischung aus Weichschaumstoffen mit dem erfindungsgemäßen Verfahren im 11,5 Liter Büchi-Reaktor behandelt. Die Mischung enthielt folgende Weichschaumqualitäten (bezogen von der Neveon Holding GmbH, Ebersbach-Fils, Deutschland):

**Tabelle 10:**

| Weichschaum | Klasse | Menge [g] |
|---|---|---|
| N 3045 | TDI 80 - Standard | 100 |
| R 4040 | TDI 80 - high resilience | 100 |
| R 5535 | MDI - high resilience | 100 |
| V 5018 | MDI - Viscoelastisch | 100 |

Die 400 Gramm Schaumstoffmischung wurden mit 236 Milliliter einer 3%igen Wasser-Harnstofflösung vorbefeuchtet und in ein Edelstahl-Aufschluss-Gefäß (Volumen ca. 10,2 Liter) eingefüllt. Der Boden des Gefäßes wurde mit Aluminiumfolie mit einer Randhöhe von 5 cm ausgekleidet, um das entstehende flüssige Verfahrensmedium aufzufangen. Unter das Inliner-Gefäß wurden 200 ml Wasser zur schnellen Erzeugung von Wasserdampf gegeben.

Das Verfahren wurde über einen Zeitraum von 240 Min durchgeführt, wobei die Reaktor-Manteltemperatur zunächst 60 Min auf 260°C gesetzt war und danach 180 Min auf 250°C. Der Reaktorinnenraum erreichte dabei am Ende eine Temperatur von knapp 236°C.

Das gewonnene Verfahrensmedium wurde wiederum über gekoppelte TG/STA-GC-MS-Messungen analysiert.

Das Spektrum bei 379°C wurde mit AMDIS und der im vorbereitenden Schritt erstellten Referenzdatenbank mit einer Voreinstellung eines Mindest- Matchfaktors (MF) von 80% untersucht. Der Matchfaktor bedeutet die Übereinstimmung des gemessen Spektrums mit den Spektren aus den AMDIS Datenbanken in Prozent. Dieser Faktor wird durch in der Software hinterlegte Algorithmen berechnet. Die Hauptpeaks entsprechen dabei den Peaks, die bei den Einzel-Polyolen laut Tabelle 9 gemessen wurden.

Die Korrelation dieser Daten ist in Tabelle 11 dargestellt. Das Analyseergebnis zeigt, dass das Verfahrensmedium ursprünglich eingesetzte Polyole sowie im Rahmen des Abbauverfahrens entstandene Umbauprodukte und/oder Abbauprodukte umfasst.

**Tabelle 11:**

| Retentionszeit [min] | Substanz | MF |
|---|---|---|
| 2.4464 | Ethanol, 2-methoxy-, acetate _**STD**_**POLYOL** (CAS#:110-49-6) | 83 |
| | IUPAC: 2-methoxyethyl acetate | |
| 3.9042 | 1-Propene, 2-(1-methylethoxy) **_HR_6000** (CAS#:4188-63-0) | 93 |
| | IUPAC: (*E*)-1-propan-2-yloxyprop-1-ene | |
| 6.5138 | Diisopropylether_HR_SAN (CAS#: 108-20-3) | 99 |
| 6.9436 | 2-Propanone, 1-(1-methylethoxy)-**_STD_POLYOL_HR_HF_VE8420** ( CAS#:42781-12-4) | 100 |
| | 1-Isopropoxyacetone | |
| | IUPAC: 1-propan-2-yloxypropan-2-one | |
| 7.4102 | Oxirane, trimethyl- **_HR_6000** (CAS#:5076-19-7) | 98 |
| | Trimethyloxiran | |
| | IUPAC: 2,2,3-trimethyloxirane | |
| 7.6279 | Carbonic acid, allyl 2-ethoxyethyl ester**_HR_6000** (NIST#: 357377) (ID#:2-87-3) | 93 |
| 8.3387 | Styrene **_SAN_45%HR_SAN** (CAS#:100-42-5) Styrol | 97 |
| 9.4547 | Diisopropyl ether **_HR_6000_HR_HF** (CAS#:108-20-3) | 99 |
| 9.5666 | 3-Pentanol, 2-methyl- **_HR_6000_HR_HF** (CAS#:565-67-3) | 98 |
| | IUPAC: 2-methylpentan-3-ol | |
| 9.9325 | 2-Pentanone, 5-methoxy- _**STD_POLYOL**_**HR_HF_VE8420** (CAS#: 17429-04-8) IUPAC: 5-methoxypentan-2-one | 89 |
| 10.1310 | Ethanol, 2-(2-ethoxyethoxy)- **_HYPERSOFT** (CAS#:111-90-0) | 98 |
| | Diethylene glycol monoethyl ether | |
| 11.3633 | 1-Propanol, 3-[3-(1-methylethoxy)propoxy]-**_STD_POLYOL_HR_SAN_HR_HF** (CAS#:54518-03-5) | 99 |
| | IUPAC: 3-(3-Isopropoxypropoxy)propan-1-ol | |
| 11.4137 | 1-Propanol, 2-(2-hydroxypropoxy)- **_VE8420** (CAS#:106-62-7) | 94 |
| | IUPAC: 2-(2-hydroxypropoxy)propan-1-ol | |
| 11.5686 | Propane, 1,1-dipropoxy-**_STD_POLYOL_HR_HF** (CAS#:4744-11-0) | 98 |
| | Dipropylpropylal | |
| 11.9289 | 1-Butoxypropan-2-yl isobutyl carbonate **_HR_6000_HR_HF** (NIST#:378276) (ID#:26899) | 98 |
| 13.0548 | 1-Propanol, 2,2'-oxybis- **_STD_POLYOL_VE8420** (CAS#:108-61-2) | 96 |
| | 2,2'-Oxydipropanol | |
| 13.2251 | Ethanol, 2-[2-(2-ethoxyethoxy)ethoxy]- **_HR_6000_HR_HF** (CAS#:112-50-5) | 83 |
| | Triethylene Glycol Monoethyl Ether | |
| 13.3345 | 12-Crown-4 _HR6000_VE8420 (CAS#:294-93-9) | 94 |
| | 1,4,7,10-Tetraoxacyclododecan | |
| 13.9039 | Diethyl carbitol**_HYPERSOFT** (CAS#:112-36-7) | 83 |
| | Diethylenglycoldiethylether | |
| 14.2187 | Tri(propylene glycol) propyl ether**_STD_POLYOL** (CAS#:96077-04-2) | 81 |
| 14.5563 | 2-Propanol, 1-[1-methyl-2-(2-propenyloxy)ethoxy]-**_STD_POLYOL_VE8420** (#:55956-25-7) | 94 |
| 15.5887 | Tripropylene glycol monomethyl ether_STD_POLYOL_VE8420 ( CAS#:20324-33-8) | 81 |
| 16.5011 | Benzene, 1,1'-(1,3-propanediyl)bis- **_SAN_45%** (CAS#:1081-75-0) | 89 |
| | 1,3-Diphenylpropan | |
| 17.6774 | 4,8,12,16-tetraoxaeicosan-1-ol **_HR_6000_HR_HF** (NIST#:397636) (ID#:32-16-4) | 96 |
| | IUPAC: 3-[3-[3-(3-butoxypropoxy)propoxy]propoxy]propan-1-ol | |
| 17.8010 | Pentaethylene glycol **_HR_SAN** (CAS#:4792-15-8) | 92 |
| 19.5699 | 3,6,9,12-Tetraoxatetradecan-1-ol **_HYPERSOFT** (CAS#:5274-68-0) | 85 |
| | IUPAC: 2-[2-[2-(2-dodecoxyethoxy)ethoxy]ethoxy]ethanol | |
| | Tetraethyleneglycol monododecyl ether | |
| 19.5699 | Tetraethylene glycol diethyl ether **_HYPERSOFT** (CAS#:4353-28-0) | 85 |
| | IUPAC: 1-ethoxy-2-[2-[2-(2-ethoxyethoxy)ethoxy]ethoxy]ethane 3,6,9,12,15-Pentaoxaheptadecane | |
| 19.5988 | 1,4,7,10,13,16-Hexaoxacyclooctadecane **_HYPERSOFT_HR_6000** ( CAS#: 17455-13-9) 18-Crown-6 ether | 84 |
| 19.6542 | 3-Phenylpropanol **_SAN_45%** (CAS#:122-97-4) | 81 |
| | IUPAC: 3-phenylpropan-1-ol | |
| | Benzenepropanol | |
| 19.9315 | (1-Benzyl-2-O-tolyl-ethyl)-isonitrile **_SAN_45%** (NIST#:287385), (ID#:88-42-6) | 82 |
| | IUPAC: *N*-[1-(2-methylphenyl)-3-phenylpropan-2-yl]methanimine | |

Das Spektrum bei 265°C wurde mit AMDIS und der Datenbanken von AMDIS und NIST mit einer Voreinstellung des Matchfaktors (MF) von 90% untersucht. In Vorversuchen war ermittelt worden, dass bei einer Temperatur von 265°C die im Verfahrensmedium enthaltenen Komponenten, die im Polyurethan auf das Diisocyanat zurückzuführen sind, thermisch abgebaut werden, wohingegen im Polyurethan auf die Polyole zurückzuführende Komponenten bei dieser Temperatur nicht abgebaut werden.

Die Hauptpeaks entsprechen den Diamin-Vorstufen der in den Weichschaumstoffen eingesetzten Di-Isocyanate (Tabelle 12). Das nach Durchführung des erfindungsgemäßen Verfahrens erhaltene flüssige Verfahrensmedium enthält somit die Vorstufen vor dem industriellen Phosgenierungs-Schritt der Di-isocyanate.

**Tabelle 12:**

| Retentionszeit [min] | Substanz | MF |
|---|---|---|
| 9.7657 | Aniline | 99 |
| 14.2512 | 1,3-Benzenediamine, 4-methyl- | 98 |
| 14.2727 | 1,3-Benzenediamine, 4-methyl- | 98 |
| 18.9949 | Benzenamine, 4,4'-methylenebis- | 92 |
| 19.4296 | Benzenamine, 4,4'-methylenebis- | 100 |
| 19.8471 | Benzenamine, 4,4'-methylenebis- | 98 |

Das beschriebene Verfahren ermöglicht den Abbau von Polyurethan und ist somit gewerblich anwendbar. Weiterhin lassen sich aus dem erhaltenen Verfahrensmedium Rohstoffe gewinnen, die erneut in der chemischen Industrie, insbesondere der Polyurethan-Kreislaufwirtschaft einsetzbar sind.

## Patentansprüche

1. Verfahren zum Abbau von Polyurethan, wobei man ein Polyurethan enthaltendes Material in Gegenwart einer 1 bis 45 Massenprozent Harnstoff enthaltenden wässrigen Lösung bei Überdruck auf eine Temperatur von 190°C bis 250°C erhitzt.

2. Verfahren nach Anspruch 1, **dadurch gekennzeichnet, dass** das Erhitzen bei einem Druck von 1,05 bar bis 100 bar erfolgt.

3. Verfahren nach einem der vorhergehenden Ansprüche, **dadurch gekennzeichnet, dass** der Überdruck ein sich bei einem Erhitzen einstellender Gleichgewichtsdruck ist.

4. Verfahren nach einem der vorhergehenden Ansprüche, **dadurch gekennzeichnet, dass** das Erhitzen über einen Zeitraum von 20 Minuten bis 240 Minuten erfolgt.

5. Verfahren nach einem der vorhergehenden Ansprüche, **dadurch gekennzeichnet, dass** die wässrige Lösung 2,5 - 10 Massenprozent Harnstoff umfasst und das Erhitzen über einen Zeitraum von 45 - 250 min erfolgt, insbesondere über einen Zeitraum von 45 bis 240 min.

6. Verfahren nach einem der vorhergehenden Ansprüche, **dadurch gekennzeichnet, dass** das Verhältnis zwischen Harnstoff enthaltender wässriger Lösung und Polyurethan enthaltendem Material 0,4 ml/g bis 5 ml/g beträgt.

7. Verfahren nach einem der vorhergehenden Ansprüche, **dadurch gekennzeichnet, dass** nach dem Erhitzen das erhaltene flüssige Verfahrensmedium wenigstens teilweise gewonnen wird.

8. Verfahren nach Anspruch 7, **dadurch gekennzeichnet, dass** aus dem gewonnenen flüssigen Verfahrensmedium Feststoffe entfernt werden.

9. Verfahren nach einem der vorhergehenden Ansprüche, **dadurch gekennzeichnet, dass** die wässrige Lösung Polyol-frei und/oder Carbonsäure-frei und/oder Ammoniak-frei ist.

10. Verfahren nach einem der vorhergehenden Ansprüche, **dadurch gekennzeichnet, dass** die wässrige Lösung aus Wasser und Harnstoff besteht.

11. Flüssiges Verfahrensmedium, erhältlich durch ein Verfahren nach einem der vorhergehenden Ansprüche, wobei das Erhitzen bei einer Temperatur von 190°C bis 250°C über einen Zeitraum von 20 Minuten bis 240 Minuten erfolgt, wobei die wässrige Lösung 1 bis 10 Massenprozent Harnstoff enthält und das Verhältnis zwischen Harnstoff enthaltender wässriger Lösung und Polyurethan enthaltendem Material 0,4 ml/g bis 5 ml/g beträgt.

## Claims

1. Process for the decomposition of polyurethane, wherein a polyurethane containing material is heated at excess pressure to a temperature of 190°C to 250°C in the presence of an aqueous solution containing 1 to 45 mass percent urea.

2. Process according to claim 1, wherein the heating is performed at a pressure of 1.05 bar to 100 bar.

3. Process according to any one of the preceding claims, wherein the excess pressure is an equilibrium pressure resulting from heating.

4. Process according to any one of the preceding claims, wherein the heating is performed for 20 minutes to 240 minutes.

5. Process according to any one of the preceding claims, wherein the aqueous solution comprises 2.5 - 10 mass percent urea and wherein the heating is performed for 45 - 250 min, in particular for 45 - 240 min.

6. Process according to any one of the preceding claims, wherein the ratio between urea containing aqueous solution and polyurethane containing material is 0.4 ml/g to 5 ml/g.

7. Process according to any one of the preceding claims, wherein the liquid process medium obtained after heating is at least partially recovered.

8. Process according to claim 7, wherein solids are removed from the recovered aqueous process medium.

9. Process according to any one of the preceding claims, wherein the aqueous solution is polyol-free and/or carboxylic acid-free and/or ammonia-free.

10. Process according to any one of the preceding claims, wherein the aqueous solution consists of water and urea.

11. Liquid process medium, obtainable by a process according to one of the preceding claims, wherein the heating is performed at a temperature of 190°C to 250°C for 20 minutes to 240 minutes, wherein the aqueous solution contains 1 to 10 mass percent urea and wherein the ratio between urea containing aqueous solution and polyurethane containing material is 0.4 ml/g to 5 ml/g.

## Revendications

1. Procédé de dégradation de polyuréthane, consistant à réchauffer un matériau contenant du polyuréthane en présence d'une solution aqueuse contenant 1 à 45 pour cent en masse d'urée à une température comprise entre 190 °C et 250 °C en appliquant une surpression.

2. Procédé selon la revendication 1, **caractérisé en ce que** réchauffement est réalisé à une pression comprise entre 1,05 bar et 100 bar.

3. Procédé selon l'une des revendications précédentes, **caractérisé en ce que** la surpression est une pression d'équilibre qui s'établit lors d'un réchauffement.

4. Procédé selon l'une des revendications précédentes, **caractérisé en ce que** le réchauffement est réalisé durant une période comprise entre 20 minutes et 240 minutes.

5. Procédé selon l'une des revendications précédentes, **caractérisé en ce que** la solution aqueuse comprend 2,5 à 10 pour cent en masse d'urée et le réchauffement est réalisé durant une période comprise entre 45 et 250 min, notamment durant une période comprise entre 45 et 240 min.

6. Procédé selon l'une des revendications précédentes, **caractérisé en ce que** le rapport entre la solution aqueuse contenant de l'urée et le matériau contenant du polyuréthane est compris entre 0,4 mL/g et 5 mL/g.

7. Procédé selon l'une des revendications précédentes, **caractérisé en ce que** le milieu de procédé liquide, obtenu suite au réchauffement, est au moins partiellement récupéré.

8. Procédé selon la revendication 7, **caractérisé en ce que** l'on retire des matières solides du milieu de procédé liquide obtenu.

9. Procédé selon l'une des revendications précédentes, **caractérisé en ce que** la solution aqueuse est exempte de polyols et/ou exempte d'acides carboxyliques et/ou exempte d'ammoniac.

10. Procédé selon l'une des revendications précédentes, **caractérisé en ce que** la solution aqueuse est constituée d'eau et d'urée.

11. Milieu de procédé liquide, pouvant être obtenu par un procédé selon l'une des revendications précédentes, le réchauffement étant réalisé à une température comprise entre 190 °C et 250 °C pendant une période comprise entre 20 minutes et 240 minutes, la solution aqueuse contenant 1 à 10 pour cent en masse d'urée et le rapport entre la solution aqueuse contenant de l'urée et le matériau contenant du polyuréthane étant compris entre 0,4 mL/g et 5 mL/g.
